# EUROPEAN PATENT APPLICATION

(11) **EP 4 563 559 A1**
(43) Date of publication of application: **04.06.2025**
(21) Application number: 23846603.1
(22) Date of filing: 27.07.2023
(51) Int. Cl.: C07B 45/00, C07F 9/165, C07F 9/6561, C07H 21/00, C12N 15/11

(54) **THIONIZING SOLUTION**

(30) Priority: 28.07.2022 JP 2022120815
(71) Applicant: Sumitomo Chemical Company, Limited, Tokyo 103-6020 (JP)
(72) Inventor: KANO, Toshifumi, Osaka-shi, Osaka 555-0021 (JP); TANAKA, Yuki, Oita-shi, Oita 870-0106 (JP); KAWAI, Hayato, Oita-shi, Oita 870-0106 (JP)
(74) Representative: Mewburn Ellis LLP
(86) International application number: PCT/JP2023/027486
(87) International publication number: WO 2024/024873

(57) **Abstract**

The present invention provides a thiolation solution with high stability, which contains a compound represented by formula (3) as a thiolating agent, and a production method of a nucleic acid molecule by the amidite method using the thiolation solution. The present invention also provides a composition which comprises an aromatic heterocyclic compound represented by formula (1) (wherein, X₁ to X₅ are identical to or different from each other and each independently represents a hydrogen atom or the like.), an aromatic hydrocarbon compound represented by formula (2) (wherein, Y₁ to Y₆ are identical to or different from each other and each independently represents a hydrogen atom, a C1-C5 alkyl group, or a halogen atom.), and a thiolating agent represented by formula (3).

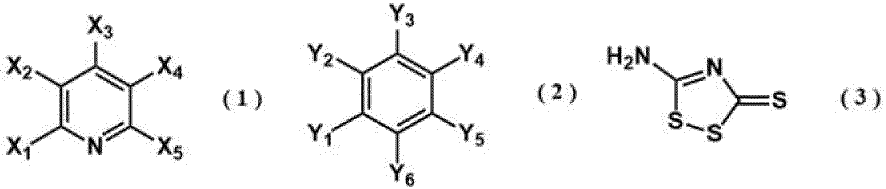

## Description

### TECHNICAL FIELD

This application claims priority to and the benefit of Japanese Patent Application No. 2022-120815 filed July 28, 2022 according to the Paris Convention, the entire contents of which are incorporated herein by reference.

The present invention relates to a thiolation solution with high stability and a production method of a nucleic acid molecule by the amidite method using the thiolation solution.

### BACKGROUND ART

In recent years, there has been increasing interest in the application of nucleic acid molecules in the medical field. For example, antisense nucleic acids, aptamers, ribozymes, siRNA, and nucleic acids that induce genome editing such as gRNA, can be mentioned.

Nucleic acid molecules can be synthesized by solid-phase synthesis method, in which a phosphoramidite (hereinafter also referred to as an "amidite") of a nucleoside is used as a raw material. In solid-phase synthesis of nucleic acid molecules using amidites, it is known that a phosphite triester generated by the coupling reaction is converted into a phosphodiester bond or a phosphorothioate bond by an oxidizing solution or a thiolation solution. Thiolating agents used in thiolation solutions generally include phenylacetyl disulfide (PADS), 3-amino-1,2,4-dithiazole-5-thione (ADTT), [(N,N-dimethylaminomethylidene)amino]-3H-1,2,4-dithiazoline-3-thione (DDTT), 3H-1,2-benzodithiol-3-one-1,1-dioxide (Beaucage reagent), 5-phenyl-3H-1,2,4-dithiazole-3-one (POS), and [(N,N-dimethylaminomethylidene)amino]-3H-1,2,4-dithiazoline-3-thione (DTD), and the others (Non-Patent Literature 1). Furthermore, as described above, thiolating agents are typically used in reactions in a solution state, but among the thiolating agents, ADTT, which is a thiolating agent, represented by the formula (3), 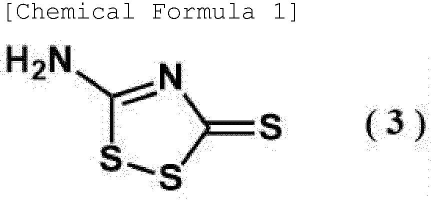 was not considered to have high stability in a solution state (composition state). Therefore, a stable thiolation solution containing ADTT has been desired.

### CITATION LIST

### NON-PATENT LITERATURE

Non-Patent Literature 1: Current protocols in nucleic acid chemistry 46.1 (2011) 4.1

### SUMMARY OF THE INVENTION

### PROBLEMS TO BE SOLVED BY INVENTION

The present invention aims to provide a thiolation solution with high stability, which contains a compound represented by formula (3) (i.e., ADTT) as a thiolating agent, and a production method of a nucleic acid molecule by the amidite method using the thiolation solution.

### MEANS TO SOLVE PROBLEMS

The present inventors, through extensive research to achieve the above-mentioned objective, have found that a thiolation solution containing a compound represented by formula (3), an aromatic heterocyclic compound, and an aromatic hydrocarbon compound has high stability. The present invention provides a thiolation solution with high stability, characterized by containing a compound represented by formula (3), an aromatic heterocyclic compound, and an aromatic hydrocarbon compound. Additionally, it provides an efficient production method of a nucleic acid molecule using the thiolation solution.

The present invention encompasses the following embodiments but are not limited thereto.
1. A composition comprising:
   an aromatic heterocyclic compound represented by formula (1) : 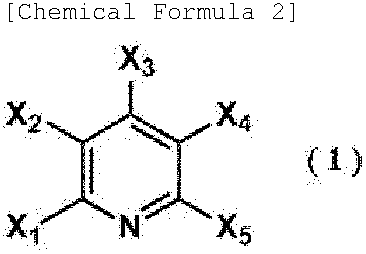 (wherein, X₁ to X₅ are identical to or different from each other and each independently represents a hydrogen atom or a C1-C5 alkyl group.),
   an aromatic hydrocarbon compound represented by formula (2): 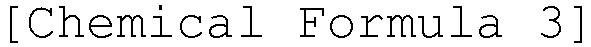 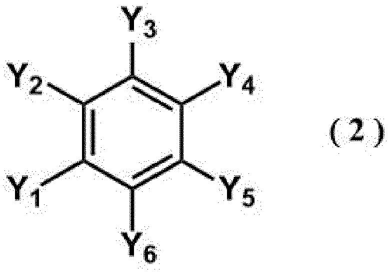 (wherein, Y₁ to Y₆ are identical to or different from each other and each independently represents a hydrogen atom, a C1-C5 alkyl group, or a halogen atom.), and
   a thiolating agent represented by formula (3): 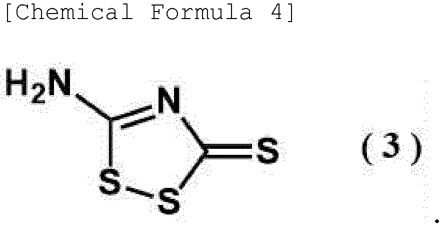
2. The composition according to [1], wherein the aromatic heterocyclic compound is pyridine, 2-methylpyridine, 3-methylpyridine, 4-methylpyridine, 2,6-lutidine, 3,4-lutidine, 3,5-lutidine, 2,3-lutidine, 2,4-lutidine, or 2,5-lutidine.
3. The composition according to [1], wherein the aromatic heterocyclic compound is pyridine or 2,6-lutidine.
4. The composition according to any one of [1] to [3], wherein the aromatic hydrocarbon compound is benzene, toluene, o-xylene, m-xylene, p-xylene, chlorobenzene, o-dichlorobenzene, m-dichlorobenzene, or p-dichlorobenzene.
5. The composition according to any one of [1] to [4], wherein the aromatic hydrocarbon compound is toluene, o-xylene, or o-dichlorobenzene.
6. The composition according to any one of [1] to [5], wherein the aromatic heterocyclic compound is pyridine.
7. The composition according to any one of [1] to [6], wherein the aromatic hydrocarbon compound is toluene.
8. The composition according to any one of [1] to [7], wherein the volume ratio of the aromatic heterocyclic compound to the aromatic hydrocarbon compound is 1:99 to 99:1.
9. The composition according to any one of [1] to [7], wherein the volume ratio of the aromatic heterocyclic compound to the aromatic hydrocarbon compound is 1:4 to 4:1.
10. The composition according to any one of [1] to [7], wherein the volume ratio of the aromatic heterocyclic compound to the aromatic hydrocarbon compound is 2:1 to 1:2.
11. The composition according to any one of [1] to [10], wherein the concentration of the thiolating agent represented by formula (3) in the composition is 0.001 to 0.3 M.
12. A production method of a nucleic acid molecule by the amidite method, comprising a step of converting a phosphite triester bond to a thiophosphate triester bond using the composition according to any one of [1] to [11].
13. A production method of a nucleic acid molecule comprising a step of contacting a precursor represented by formula (4): 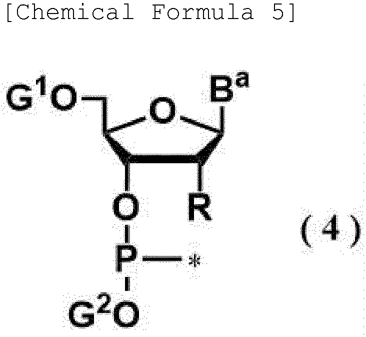 (wherein,
   G¹ and G² are identical to or different from each other and each independently represents a protecting group of a hydroxy group,
   B^{a} represents a nucleobase which may be protected by a protecting group,
   R represents a protected hydroxy group, a hydrogen atom, a fluorine atom, a methoxy group, a 2-methoxyethyl group, an OQ' group, or an NQ' group,
   Q' represents an alkylene group or a carbonyl group, which are bonded to the carbon atom at the 4' position of the ribose, and,
   the bond marked with * indicates the binding position to the nucleotide unit at the 3' terminal side.) which has a phosphite triester bond with the composition according to any one of [1] to [12] to convert into a nucleic acid compound represented by formula (5): 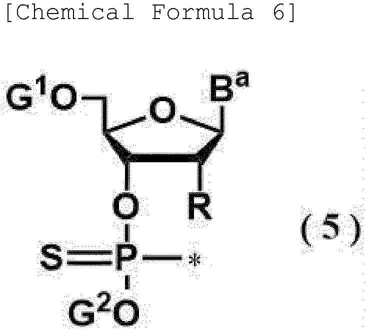 (wherein, the symbols have the same meanings as described above.)
      which has a thiophosphate triester bond.
14. The production method of the nucleic acid molecule according to [13], wherein the precursor which has the phosphite triester bond is a compound represented by formula (6) : 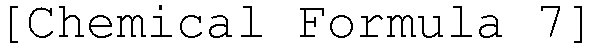 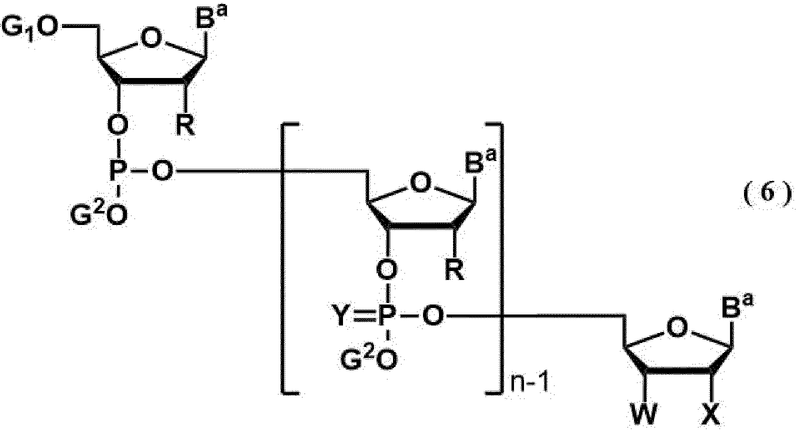 (wherein,
   G¹ represents a protecting group of a hydroxy group,
   G² is identical to or different from each other and each independently represents a protecting group of a hydroxy group,
   B^{a} is identical to or different from each other and each independently represents a nucleobase which may be protected by a protecting group,
   R is identical to or different from each other and each independently represents a protected hydroxy group, a hydrogen atom, a fluorine atom, a methoxy group, a 2-methoxyethyl group, an OQ' group, or an NQ' group,
   Q' is identical to or different from each other and each independently represents an alkylene group or a carbonyl group, which are bonded to the carbon atom at the 4' position of the ribose,
   Y is identical to or different from each other and each independently represents an oxygen atom or a sulfur atom,
   n represents any integer from 1 to 300,
   when X represents an OZ group, W represents an OV group, and V represents a protecting group of a hydroxy group, or,
   when X represents an R group, W represents an OZ group, and,
   Z is a group having a structure consisting of a solid support and a connecting group.
   And, the compound which has the thiophosphate triester bond is a nucleic acid compound represented by formula (7): 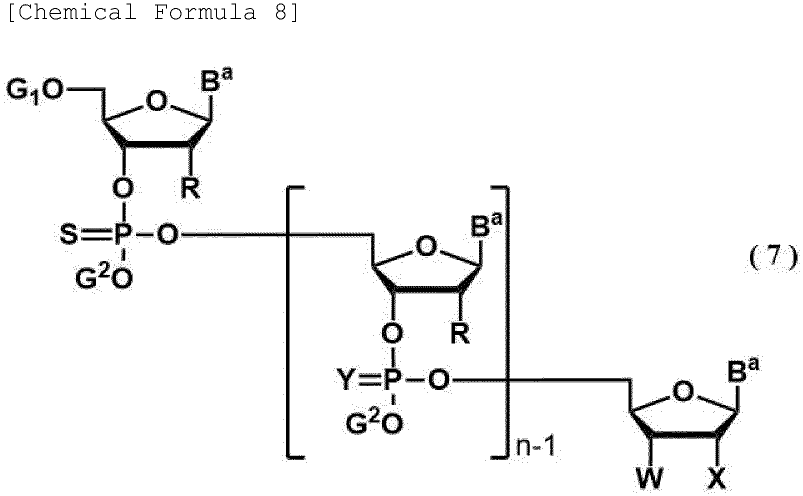 (wherein, the symbols have the same meanings as described above.) .
15. The production method of the nucleic acid molecule according to [13] or [14], wherein the precursor which has the phosphite triester bond is a compound represented by formula (8): 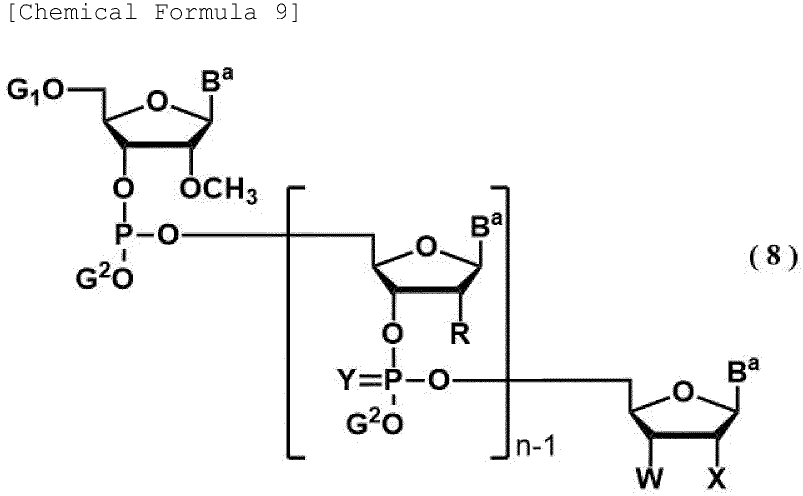 (wherein,
   G¹ represents a protecting group of a hydroxy group,
   G² is identical to or different from each other and each independently represents a protecting group of a hydroxy group,
   B^{a} is identical to or different from each other and each independently represents a nucleobase which may be protected by a protecting group,
   R is identical to or different from each other and each independently represents a protected hydroxy group, a hydrogen atom, a fluorine atom, a methoxy group, a 2-methoxyethyl group, an OQ' group, or an NQ' group,
   Q' is identical to or different from each other and each independently represents an alkylene group or a carbonyl group, which are bonded to the carbon atom at the 4' position of the ribose,
   Y is identical to or different from each other and each independently represents an oxygen atom or a sulfur atom,
   n represents any integer from 1 to 300,
   when X represents an OZ group, W represents an OV group, and V represents a protecting group of a hydroxy group, or,
   when X represents an R group, W represents an OZ group, and,
   Z is a group having a structure consisting of a solid support and a connecting group.), and, the compound which has the thiophosphate triester bond is a nucleic acid compound represented by formula (9): 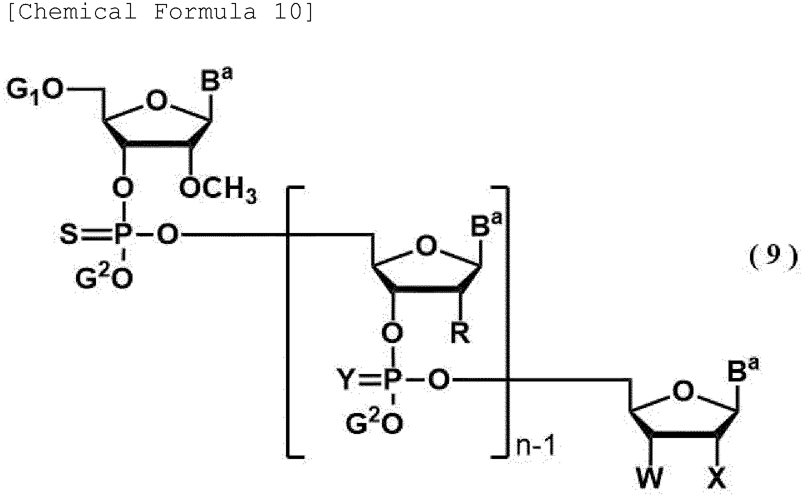 (wherein, the symbols have the same meanings as described above.) .

### Effect of Invention

The present invention provides a thiolation solution with high stability and a production method of a nucleic acid molecule by the amidite method using the thiolation solution. The present invention is expected to improve the stability of a thiolation solution. Additionally, the present invention is expected to improve the purity of the nucleic acid molecule produced.

### BRIEF DESCRIPTION OF THE DRAWINGS

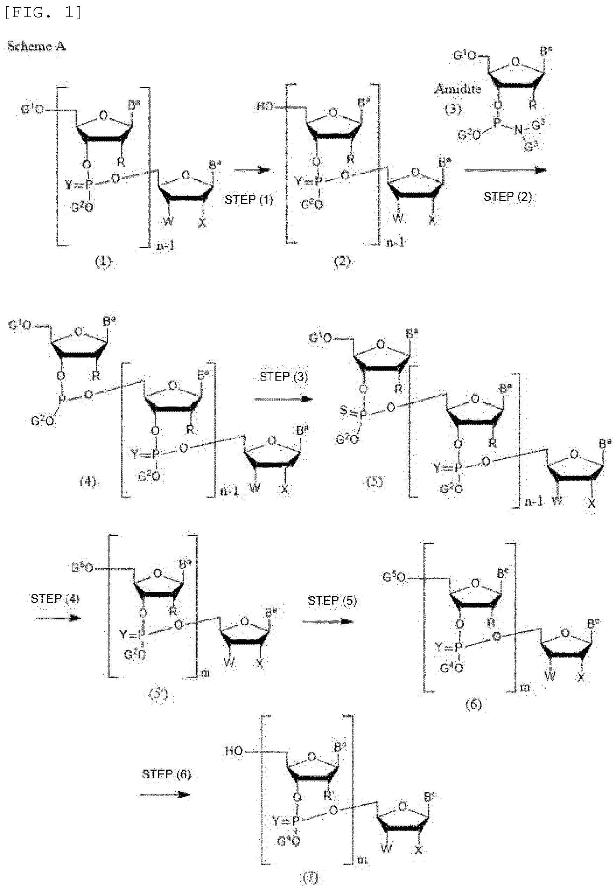

[Figure 1] Figure 1 is a drawing showing the scheme (Scheme A) of steps (1) to (6) of the production method of the present invention.

### MODE FOR CARRYING OUT THE INVENTION

The thiolation solution of the present invention relates to a composition comprising an aromatic heterocyclic compound represented by formula (1): 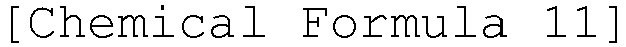 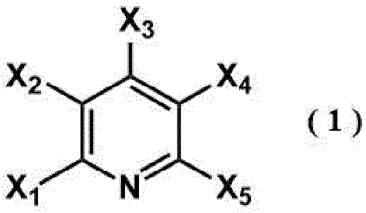 (wherein, X₁ to X₅ are identical to or different from each other and each independently represents a hydrogen atom or a C1-C5 alkyl group.),
an aromatic hydrocarbon compound represented by formula (2): 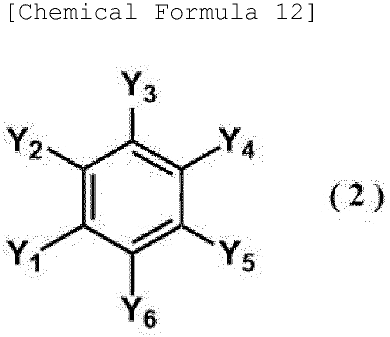 (wherein, Y₁ to Y₆ are identical to or different from each other and each independently represents a hydrogen atom, a C1-C5 alkyl group, or a halogen atom.), and
a thiolating agent represented by formula (3):

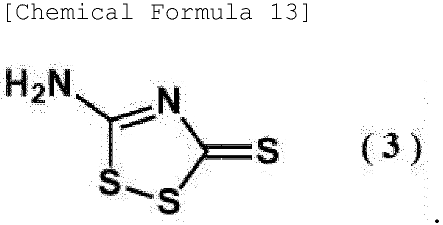

A composition comprising an aromatic heterocyclic compound, an aromatic hydrocarbon compound, and a compound represented by formula (3) used as a thiolating agent (this compound is named "3-amino-1,2,4-dithiazole-5-thione" (hereinafter abbreviated as "ADTT" in this description)) is described here.

The composition can be used as a thiolating agent in this description and is also referred to as a "thiolation solution."

In the composition of the present invention, examples of the aromatic heterocyclic compounds that can be used include pyridine, 2-methylpyridine, 3-methylpyridine, 4-methylpyridine, 2,6-lutidine, 3,4-lutidine, 3,5-lutidine, 2,3-lutidine, 2,4-lutidine, and 2,5-lutidine, but are not limited to these. Preferably, the aromatic heterocyclic compound is pyridine or 2,6-lutidine, and more preferably, pyridine.

Examples of the aromatic hydrocarbon compounds include benzene, toluene, o-xylene, m-xylene, p-xylene, monochlorobenzene, o-dichlorobenzene, m-dichlorobenzene, and p-dichlorobenzene, but are not limited to these. Preferably, the aromatic hydrocarbon compound is toluene, o-xylene, or o-dichlorobenzene, and more preferably, toluene.

The volume ratio of the aromatic heterocyclic compound to the aromatic hydrocarbon compound contained in the composition of the present invention can be any ratio. For example, compositions where the volume ratio of the aromatic heterocyclic compound to the aromatic hydrocarbon compound is 9:1, 4:1, 2:1, 1:1, 1:2, 1:4, or 1:9 can be exemplified, preferably, 1-99:1-99, more preferably 20-80:20-80 (i.e., 1:4 to 4:1), and even more preferably 40-80:40-80 (i.e., 1:2 to 2:1).

The concentration of the compound represented by formula (3) contained in the composition of the present invention is usually 0.001M to 0.3M, but it is not particularly limited as long as it is an effective concentration for the synthesis of nucleic acid molecules. Preferably, it is 0.001M to 0.2M, more preferably 0.003M to 0.1M, and even more preferably 0.005M to 0.05M.

The water content in the composition of the present invention is usually 0.001wt% to 0.2wt%, preferably 0.001wt% to 0.1wt%, and more preferably 0.001wt% to 0.05wt%.

The analytical method for the compound represented by formula (3) contained in the thiolation solution is described here. It is conducted by analyzing a predetermined amount of the thiolation solution sample using high-performance liquid chromatography (HPLC).

The analysis of the compound represented by formula (3) using HPLC is usually conducted using an ODS column. As a mobile phase, for example, it is conducted with a gradient using an ammonium acetate aqueous solution as mobile phase A and an ammonium acetate aqueous solution-methanol mixture as mobile phase B. The UV detection wavelength is typically 285 nm.

Next, a production method of a nucleic acid molecule by the amidite method, which comprises a step of contacting a thiolation solution containing an aromatic heterocyclic compound and an aromatic hydrocarbon compound with a precursor having a phosphite triester bond is described here.

A nucleic acid compound represented by formula (4) is exemplified as the precursor having the phosphite triester bond. 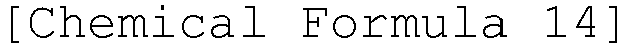 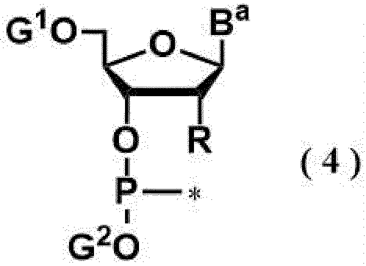 (wherein, the symbols have the same meanings as described above.)

A nucleic acid compound represented by formula (5) is exemplified as the nucleic acid compound which is produced by contacting the thiolation solution. 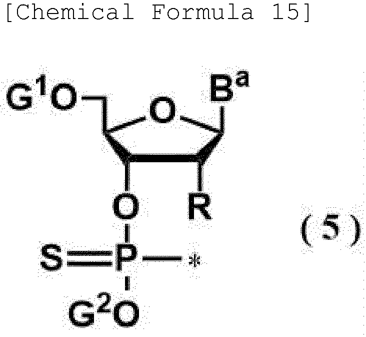 (wherein, the symbols have the same meanings as described above.)

In formulas (4) and (5), when R represents an OQ' group or an NQ' group, and Q' represents an alkylene group or a carbonyl group, which are bonded to the carbon atom at the 4' position of the ribose, specific examples of such structures include LNA-1 to LNA-7 of the following formula (10) . 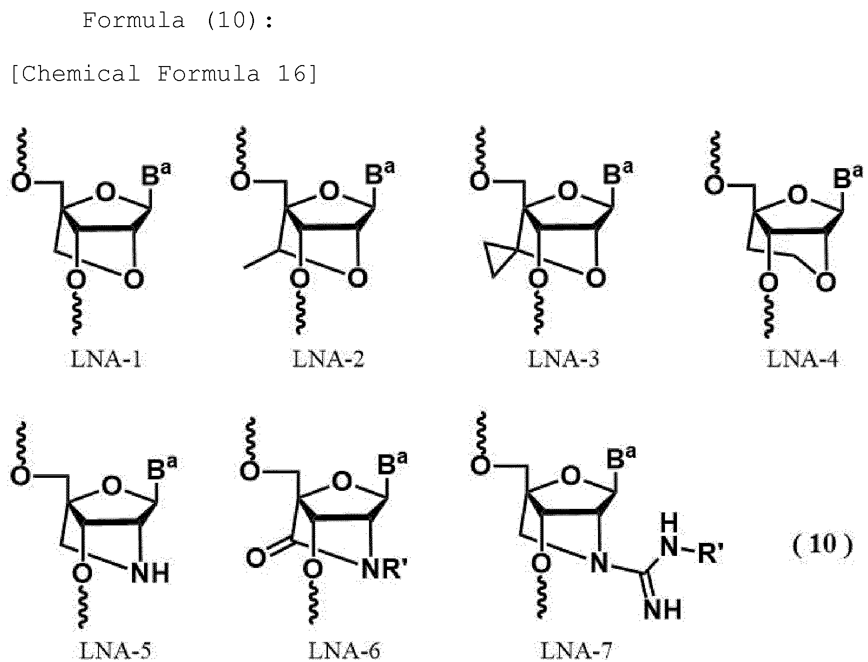 (wherein,
B^{a} represents a nucleobase which may be protected,
R' represents a hydrogen atom or a methyl group.)

The nucleotide units contained in the nucleic acid molecule used in the present invention include DNA, RNA, 2'-O-Me, 2'-F, 2'-O-MOE (2'-O-methoxyethyl), UNA, morpholino nucleic acid, and the aforementioned LNA, but are not limited to these.

As a group represented by Z, which consists of a solid support and a connection group connecting the solid support with the oxygen atom of the 2' or 3' hydroxy group at the 3' terminal ribose of the nucleic acid oligomer (it is also referred to as an "oligonucleotide"), more specifically, a structure represented by the following formula (11) can be listed. More specifically, a structure represented by the following formula (11) can be listed.

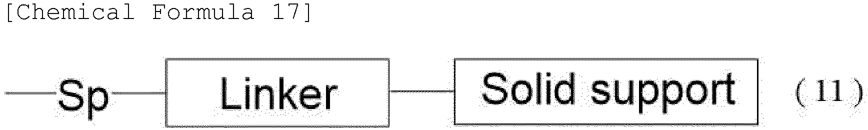

In formula (11), Sp represents a spacer.

The Spacer (Sp) is exemplified by a group having a structure represented by the following formula (12).

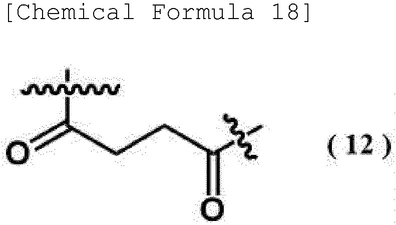

The linker may be, for example, a structure shown in the following formula (13), or a structure in which a hexamethyleneamino group portion in formula (13) is absent and an aminopropyl group is bonded to Si. Alternatively, the linker may be a structure shown in the following formula (14) . 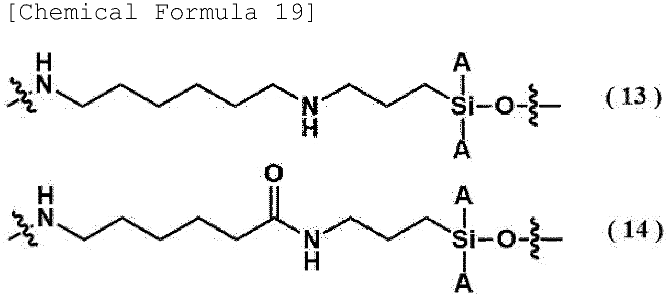 (wherein,
A may be a hydroxy group, an alkoxy group, or an alkyl group. Examples of the alkoxy group include a methoxy group and an ethoxy group. Examples of the alkyl group include a methyl group, an ethyl group, an isopropyl group, and an n-propyl group. Si indicates that it is bonded to the oxygen of a hydroxy group on the surface of a support.)

Examples of the solid support include an inorganic porous support and an organic resin support, and the others. Examples of the inorganic porous support include Controlled Pore Glass (CPG) and zeolite. Examples of the organic resin support include a support composed of polystyrene.

The step of contacting the thiolation solution can be conducted under an atmospheric atmosphere, but it is preferable to conduct it under an inert gas (e.g., nitrogen, argon) atmosphere.

The method for synthesizing a nucleic acid molecule by solid-phase synthesis method, which comprises the step of contacting the thiolation solution, typically comprises the following steps.
(1) a step of deprotecting a hydroxy group at a 5' position of a nucleoside whose hydroxy group is protected and which is bonded to a solid support via a linker;
(2) a step of coupling reaction of the hydroxy group at the 5' position produced in the previous step with an amidite to obtain a phosphite triester compound;
(3) a step of oxidizing the phosphite triester produced in the previous step to convert it into a phosphate triester to produce an elongated nucleic acid molecule, or a step of reacting with a thiolation solution to convert it into a thiophosphate triester, provided that the step of reacting with the thiolation solution to convert it into the thiophosphate triester is included at least once;
(4) a step of synthesizing a nucleic acid molecule on the solid support by repeating, any number of times, a series of reaction cycles composed of the steps (1) to (3), that is, the deprotecting step of the hydroxy group at the 5' position of the produced nucleic acid molecule, the coupling step of the hydroxy group at the 5' position with the amidite compound, and the oxidizing step of the produced phosphite triester;
(5) a step of subjecting the nucleic acid molecule on the solid support produced in the step (4) to a step of cleavage and deprotection to release it from the solid support to produce a nucleic acid molecule with the protecting group removed therefrom; and
(6) a step of deprotecting a protecting group of a hydroxy group at a 2' position of a ribose or at a 3' position of a 3' terminal, constituting a nucleic acid molecule.

However, in the method for synthesizing the nucleic acid molecule, a step of capping a hydroxy group at a 5' position where the coupling reaction with the amidite did not proceed may be included following the step (2) or (3), and a capping step may be added at any point during the series of reaction cycles constituting the step (4).

The step (5) is more specifically conducted in the order of the following reactions (5-1) and (5-2) for the nucleic acid molecule on the solid support produced in the step (4). Here, the reaction of step (5-1) may be optionally conducted, and the reaction of step (5-2) may be conducted using the method described in JP 4705716 B2. As a result, it is possible to produce a nucleic acid molecule with the protecting groups removed from the nucleic acid molecule released from the solid support, or a nucleic acid molecule with the 5' terminal hydroxy group protected.
(5-1) a reaction of deprotecting a protecting group of a hydroxy group at a 5' terminal of a nucleic acid molecule; and
(5-2) a reaction of cleaving and releasing a nucleic acid molecule from a solid support.

The step (6) is more specifically conducted by subjecting the nucleic acid molecule, which is obtained in the step (5), released from the solid support, and deprotected, to the deprotection reaction of the following step (6).

(6) a reaction of deprotecting the protecting group of the hydroxy group at the 2' position of the ribose or at the 3' position of the 3' terminal, constituting the nucleic acid molecule.

The scheme of the steps (1) to (6) is shown as Scheme A in Figure 1. The synthesis of a nucleic acid compound by the amidite method in the steps (1) to (5), except for the thiolation step related to the present invention in the step (1) or step (5) in the scheme in Figure 1, can be conducted by repeating the deprotection step and the coupling step according to generally known methods (for example, the methods described in JP 5157168 B2 or JP 5554881 B2), thereby conducting the nucleic acid elongation reaction. The following is an explanation of each step. The thiolation reaction in the step (3) or step (4) shown in Figure 1 is conducted using the aforementioned thiolation solution. Among the substituents in the chemical formulas in Scheme A, the definitions of G¹, G², B^{a}, and R are as described above. Additionally, the definitions of G³, G⁴, G⁵, B^{c}, and R' are as described later. Additionally, in the chemical formulas in Scheme A,
Y is identical to or different from each other and each independently represents an oxygen atom or a sulfur atom,
X represents an R group or an OZ group, where Z is as described above,
W represents an OZ group when X represents an R group, where Z is as described above, or W represents an OV group when X represents an OZ group, where V represents a protecting group of a hydroxy group,
W may include groups derived from the W group (for example, residues cleaved from a solid support or deprotected groups),
X may include groups derived from the X group (for example, residues cleaved from a solid support or deprotected groups),
n represents an integer from 1 to 300, and
m represents an integer from 1 to 300.

As for G¹, it can be used without any particular limitation as long as it can function as a protecting group, and publicly known protecting groups used for amidite compounds can be widely used.

G¹ is preferably the following group. 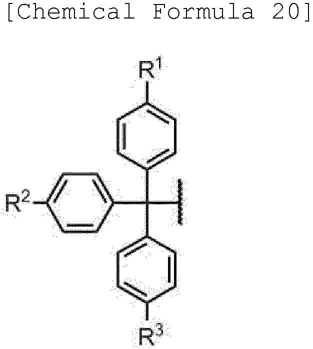 (wherein, R¹, R² and R³ are identical to or different from each other, and each independently represents a hydrogen atom or an alkoxy group.)

One of R¹, R² and R³ is a hydrogen atom, and the remaining two thereof are identical to or different from each other (preferably identical) and represents preferably an alkoxy group, and as the alkoxy group, a methoxy group is particularly preferred.

As for G², it can be used without any particular limitation as long as it can function as a protecting group, and publicly known protecting groups used for amidite compounds can be widely used. Examples of G² include an alkyl group, an alkenyl group, an alkynyl group, a cycloalkyl group, a haloalkyl group, an aryl group, a heteroaryl group, an arylalkyl group, a cycloalkenyl group, a cycloalkylalkyl group, a cyclylalkyl group, a hydroxyalkyl group, an aminoalkyl group, an alkoxyalkyl group, a heterocyclylalkenyl group, a heterocyclylalkyl group, a heteroarylalkyl group, a silyl group, a silyloxyalkyl group, a mono, di or tri- alkylsilyl group, a mono, di or tri-alkylsilyloxyalkyl group, and the others, and these groups may be substituted with one or more electron-withdrawing groups.

G² is preferably an alkyl group substituted with an electron-withdrawing group. Examples of the electron-withdrawing group include a cyano group, a nitro group, an alkylsulfonyl group, a halogen atom, an arylsulfonyl group, a trihalomethyl group, a trialkylamino group, and the others, and preferably a cyano group.

As for G², a 2-cyanoethyl group (represented by the following formula) is particularly preferred.

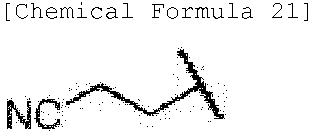

As for G³, two G³ may be combined with each other to form a cyclic structure. Preferably, both G³ are an isopropyl group.

The alkyl group as the definitions of the above R¹, R², R³, G², and G³ may be a straight chain or a branched chain, and preferably an alkyl group containing 1 to 12 carbon atoms, and more preferably an alkyl group containing 1 to 6 carbon atoms. Specific examples of the alkyl group include methyl, ethyl, n-provyl, isopropyl, n-butyl, isobutyl, tert-butyl, n-pentyl, isopentyl, and n-hexyl. An alkyl group part which constitutes the alkoxy group in the definition for above substituents has the same definition as that described in the definition of the alkyl group described here.

In this description, a nucleobase refers to a group having a natural type or non-natural type nucleobase backbone. The nucleobase also includes modified forms in which the natural type or non-natural type nucleobase backbone is modified.

A nucleobase represented by B^{a} which may be protected by a protecting group is not particularly limited. Examples of the nucleobase include adenine, cytosine, guanine, uracil, thymine, 5-methylcytosine, pseudouracil, 1-methylpseudouracil, and the others. In addition, the nucleobase may be substituted with substituent(s). Examples of the substituent include a halogen atom such as a fluoro group, a chloro group, a bromo group, and an iodo group, an acyl group such as an acetyl group, an alkyl group such as a methyl group and an ethyl group, an arylalkyl group such as a benzyl group, an alkoxy group such as a methoxy group, an alkoxyalkyl group such as a methoxyethyl group, a cyanoalkyl group such as a cyanoethyl group, a hydroxy group, a hydroxyalkyl group, an acyloxymethyl group, an amino group, a monoalkylamino group, a dialkylamino group, a carboxy group, a cyano group, a nitro group and the others, as well as combinations of two or more of these substituents.

When a nucleobase has an amino group outside the ring, a protecting group of the amino group is not particularly limited, and publicly known protecting groups used in nucleic acid chemistry can be used. Such protecting groups include, for example, a benzoyl group, a 4-methoxybenzoyl group, an acetyl group, a propionyl group, a butyryl group, an isobutyryl group, a phenylacetyl group, a phenoxyacetyl group, a 4-tert-butylphenoxyacetyl group, a 4-isopropylphenoxyacetyl group, and a (dimethylamino)methylene group and the others, as well as combinations of two or more of these protecting groups.

As the nucleobase represented by B^{a}, more specifically, the following structures are exemplified. 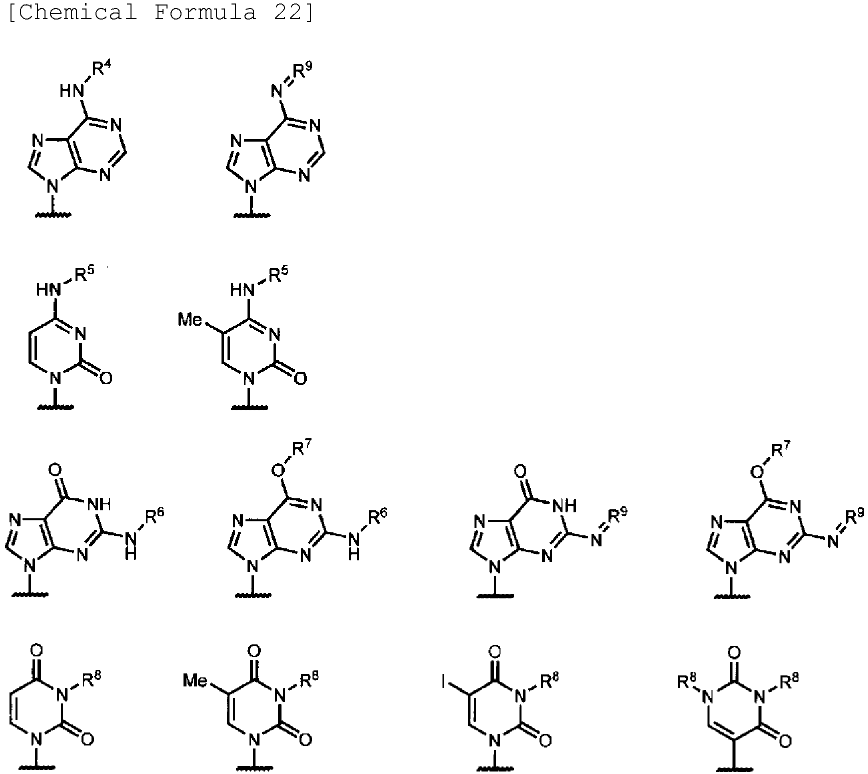 (wherein,
R⁴ represents a hydrogen atom, a methyl group, a phenoxyacetyl group, a 4-tert-butylphenoxyacetyl group, a 4-isopropylphenoxyacetyl group, a phenylacetyl group, an acetyl group, or a benzoyl group,
R⁵ represents a hydrogen atom, an acetyl group, an isobutyryl group, or a benzoyl group,
R⁶ represents a hydrogen atom, a phenoxyacetyl group, a 4-tert-butylphenoxyacetyl group, a 4-isoproylphenoxyacetyl group, a phenylacetyl group, an acetyl group or an isobutyryl group,
R⁷ represents a 2-cyanoethyl group,
R⁸ represents a hydrogen atom, a methyl group, a benzoyl group, a 4-methoxybenzoyl group, or a 4-methylbenzoyl group, and
R⁹ represents a dimethylaminomethylene group.)
It represents a group represented by any of the above.

In addition, in the method of the present invention, an amidite can be used in a free state or a salt state. Examples of the salt of the amidite include a base addition salt and an acid addition salt, but which are not particularly limited thereto. Specific examples of the base addition salt include salts with inorganic bases such as sodium salts, magnesium salts, potassium salts, calcium salts, aluminum salts and the others; salts with organic bases such as methylamine, ethylamine, ethanolamine and the others; salts with basic amino acids such as lysine, ornithine, arginine and the others; and ammonium salts. Specific examples of acid addition salts include mineral acids such as hydrochloric acid, hydrobromic acid, hydroiodic acid, sulfuric acid, nitric acid, phosphoric acid and the others; salts with organic acids such as formic acid, acetic acid, propionic acid, oxalic acid, malonic acid, malic acid, tartaric acid, fumaric acid, succinic acid, lactic acid, maleic acid, citric acid, methanesulfonic acid, trifluoromethansulfonic acid, ethanesulfonic acid and the others; and salts with acidic amino acids such as aspartic acid, glutamic acid and the others. Examples of the amidite compounds encompass salts, hydrates, solvates, crystal polymorphs, and the others.

When R represents a protected hydroxy group, the protecting group can be any that can be used in the amidite method, examples include a 2'-tert-butyldimethylsilyl (TBDMS) group, a 2'-bis(2-acetoxy)methyl (ACE) group, a 2'-(triisopropylsilyloxy)methyl (TOM) group, a 2'-(2-cyanoethoxy)ethyl (CEE) group, a 2'-(2-cyanoethoxy)methyl (CEM) group (WO 2006/022323 A1), a 2'-para-toluylsulfonylethoxymethyl (TEM) group, a 2'-EMM group (WO 2013/027843 A1), and those described in WO 2019/208571 A1. Among these 2' protecting groups for ribonucleoside (RNA), a protecting group represented by formula (15) is exemplified as a preferred protecting group. More preferably, a protecting group represented by formula (16), having a cyano group as an electron-withdrawing group represented by E_{W}, is exemplified. 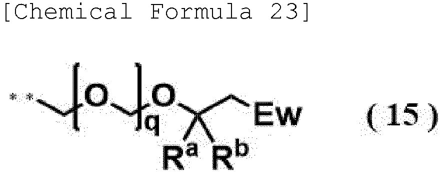 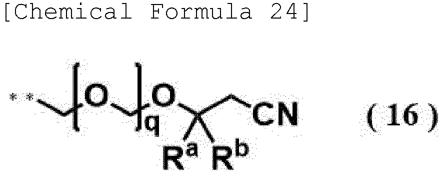 (wherein,
q represents an integer from 0 to 5,
R^{a} and R^{b} are identical to or different from each other and each represents a methyl group, an ethyl group, or a hydrogen atom,
the bond marked with ** is bonded to the oxygen of the protected hydroxy group, and
E_{W} represents an electron-withdrawing group.)

The protecting group represented by formula (16) can be synthesized according to the descriptions in WO 2013/027843 A1 and WO 2019/208571 A1, for example. Amidite compounds having the protecting group can be used for the production of nucleic acid molecules.

In the elongation reaction of nucleic acids, the amidite of formula (3) described in Scheme A of Figure 1 is used.

### (Nucleic Acid Elongation Reaction)

In this description, the "nucleic acid elongation reaction" refers to a reaction that elongates a nucleic acid molecule by sequentially connecting nucleotides via phosphodiester bonds. The nucleic acid elongation reaction can be conducted according to the general amidite method (phosphoramidite method). The nucleic acid elongation reaction may be conducted using a nucleic acid automatic synthesizer that employs the amidite method.

The chain length of the nucleic acid oligomer can be, for example, 20 mer or more, 40 mer or more, 50 mer or more, 60 mer or more, 80 mer or more, 100 mer or more, 200 mer or more, 2 to 300 mer, 2 to 250 mer, 2 to 200 mer, 10 to 300 mer, 10 to 250 mer, 10 to 200 mer, 10 to 150 mer, 15 to 300 mer, 15 to 250 mer, 15 to 200 mer, 15 to 150 mer, or 15 to 110 mer.

The 5' deprotection step in the step (1) is a step of deprotecting a protecting group of a 5' hydroxy group at RNA chain terminal which is supported on a solid support. As a general protecting group, a 4,4'-dimethoxytrityl group (DMTr group), a 4-monomethoxytrityl group, and a 4,4',4"-trimethoxytrityl group are used. Deprotection can be conducted using an acid. Examples of the acid for deprotection include trifluoroacetic acid, dichloroacetic acid, trifluoromethanesulfonic acid, trichloroacetic acid, methanesulfonic acid, hydrochloric acid, acetic acid, p-toluenesulfonic acid, and the others.

The coupling step in the step (2) is a reaction where a nucleoside phosphoramidite represented by the following formula (3) described in Scheme A of Figure 1 is attached to the 5' hydroxy group at the oligonucleotide chain terminal deprotected in the above deprotection step. Note that the amidite compound represented by formula (3) is used as the amidite for nucleic acid elongation. Additionally, other usable amidites include 2'-OMe, 2'-F, a 2'-O-tert-butyldimethylsilyl group, a 2'-O-methoxyethyl group, 2'-H, 2'-fluoro-2'-deoxy-β-D-arabinofuranosyl, and the like. As the above nucleoside amidites, those where 5' hydroxy group is protected with a protecting group (for example, DMTr group) are used. The coupling step can be conducted by using an activator which activates the above-mentioned nucleoside amidite. Examples of the activator include 5-benzylthio-1H-tetrazole (BTT), 1H-tetrazole, 4,5-dicyanoimidazole (DCI), 5-ethylthio-1H-tetrazole (ETT), N-methyl benzimidazoliumtriflate (N-MeBIT), benzimidazoliumtriflate (BIT), N-phenylimidazoliumtriflate (N-PhIMT), imidazoliumtriflate (IMT), 5-nitrobenzimidazoliumtriflate (NBT), 1-hydroxybenzotriazole (HOBT), 5-(bis-3,5-trifluoromethylphenyl)-1H-tetrazole, and the others.

The nucleoside amidite represented by formula (3) described in Scheme A of Figure 1 (hereinafter referred to as amidite) is as follows.

A compound represented by formula: 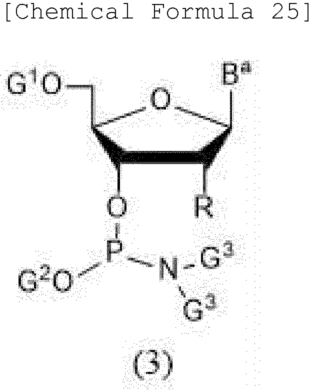 (wherein, G¹, G², G³, B^{a}, and R are as described above.) .

After the coupling step, as needed, an unreacted 5' hydroxy group may be capped. The capping can be conducted by using publicly known capping solutions such as an acetic anhydride-tetrahydrofuran solution, a phenoxyacetic anhydride/N-methylimidazole solution, and the others.

The oxidizing step in the step (3) is a step of converting a phosphite group which is formed in the above coupling step into a phosphate group or a thiophosphate group. This step is a reaction of converting a trivalent phosphorus into a pentavalent phosphorus using an oxidizing agent, which can be conducted by reacting an oxidizing agent with an oligonucleic acid derivative supported on a solid support.

When a phosphite group is converted into a phosphate group, as "oxidizing agent", for example, iodine can be used. The oxidizing agent can be prepared and used at a concentration of 0.005 to 2 M. Water can be used as the oxygen source for oxidation, and pyridine, N-methylimidazole (NMI), N-methylmorpholine, or triethylamine can be used as the base to proceed the reaction. In addition, as for solvent, it is not particularly limited as long as it does not participate in the reaction, but acetonitrile, tetrahydrofuran (THF), or a mixture of these in any ratio can be used. For example, iodine/water/pyridine/acetonitrile, iodine/water/pyridine, iodine/water/pyridine/NMI, or iodine/water/pyridine/THF can be used. The reaction temperature is preferably 5°C to 50°C. The reaction time is usually suitable between 1 minute and 30 minutes. The amount of reagent used is preferably 1 to 100 mol per 1 mol of the compound supported on the solid support, more preferably 1 to 10 mol.

When a phosphite group is converted into a thiophosphate group, the composition of the present invention is used as a "thiolating agents" that acts as an oxidizing agent. The concentration of the compound represented by formula (3) contained in the composition of the present invention is usually 0.001M to 0.3M, but it can be any concentration effective for the synthesis of nucleic acid molecules and is not particularly limited. Preferably, it is 0.001M to 0.2M, more preferably 0.003M to 0.1M, and even more preferably 0.005M to 0.05M. The volume ratio of an aromatic heterocyclic compound to an aromatic hydrocarbon compound contained in the composition of the present invention can be any ratio. For example, compositions with a volume ratio of an aromatic heterocyclic compound to an aromatic hydrocarbon compound of 9:1, 4:1, 2:1, 1:1, 1:2, 1:4, or 1:9 are exemplified. Preferably, the ratio is 1-99:1-99, more preferably 20-80:20-80 (i.e., 1:4 to 4:1), and even more preferably 40-80:40-80 (i.e., 1:2 to 2:1). The water content in the composition of the present invention is usually 0.001wt% to 0.2wt%, but preferably 0.001wt% to 0.1wt%, and more preferably 0.001wt% to 0.05wt%. The oxidizing step, including the thiolation step, may be conducted after the capping operation, or conversely, the capping operation may be conducted after the oxidizing step, and this order is not limited.

In the step (5), a step of deprotecting a phosphate protecting group involves applying an amine compound to deprotect a protecting group in the phosphate portion after the synthesis of the nucleic acid with the desired sequence is completed. Examples of the amine compound include, for example, diethylamine and the others as described in JP 4705716 B2.

The protecting group of the 5' hydroxy group of the nucleoside incorporated in the last stage of the elongation may be used for the column purification with the 5' protecting group as a tag after the below-mentioned procedures of cleaving from a solid support and deprotecting of a protecting group, or alternatively, the protecting group of the 5' hydroxy group may be deprotected after the column purification.

In the step (5), the cleavage of the nucleic acid oligomer, which has been elongated to the desired chain length on the solid support, from the solid support is typically conducted by using concentrated aqueous ammonia as a cleaving agent.

Further, using ammonia or an amine compound or the others, for example, an oligonucleotide chain is collected by cleaving from a solid support. Examples of the amine compound include methylamine, ethylamine, isopropylamine, ethylenediamine, diethylamine, and the others.

In the step (6), the protecting group of the 2' or 3' hydroxy group of the ribose in the nucleic acid compound (6) cleaved from the solid support can be removed according to the methods described in WO 2006/022323 A1, WO 2013/027843 A1, or WO 2019/208571 A1, to obtain the deprotected nucleic acid oligomer (7).

A nucleotide and an amidite wherein an R group in formula (4) is a substitute other than a hydroxy group can be produced from nucleosides which are synthesized according to known methods described in JP 3745226 B2 and so on, or WO 2001/053528 A1, JP 2014-221817 A1 or known methods referred to in these documents. Further, they can be produced by using a commercially available compound in line with the method described in the below Examples or methods with appropriate modifications to these methods.

Examples of the nucleic acid molecule which can be produced according to the production method of the present invention include those wherein a nucleoside contained in the nucleic acid molecule is RNA, DNA, RNA containing 2'-O-MOE, 2'-O-Me, or 2'-F, and LNA, which is not limited thereto. For example, various nucleosides described in Xiulong, Shen et al., Nucleic Acids Research, 2018, Vol. 46, No.46, 1584-1600, and Daniel O'Reilly et al., Nucleic Acids Research, 2019, Vol. 47, No.2, 546-558 are included. Preferably, the nucleic acid molecule produced by the method of the present invention is RNA.

As typical examples of nucleic acid molecule which can be used in the production method of the present invention, the following examples are indicated in addition to examples described in working examples, which are not limited thereto.

Hereinafter, in a description of a sequence, U represents uridine (ST.25 format), C represents cytidine, A represents adenosine, and G represents guanosine.

Nucleic acid molecules having the following sequences (A) and (B) as described in WO 2019/060442 A1 are exemplified.
Sequence (A) : 5'-AUGGAAUmACUCUUGGUUmACdTdT-3' (conforms to ST.25 format) (5'-ATGGAATmACTCTTGGTTmACdTdT-3' (conforms to ST.26 format)) (Antisense) (Sequence No. 1) 21 mer
Sequence (B): 5'-GUmAACmCmAAGAGUmAUmUmCmCmAUmdTdT (conforms to ST.25 format) (5'-GTmAACmCmAAGAGTmATmTmCmCmATmdTdT-3' (conforms to ST.26 format)) (Sense) (Sequence No. 2) 21 mer

In the sequences (A) and (B), Um represents 2'-O-methyluridine (ST.25 format), Tm represents 2'-O-methyluridine (ST.26 format), Cm represents 2'-O-methylcytidine, and dT represents thymidine. As described herein, unless stated otherwise, the abbreviations in the sequence may be applied to both of the ST.25 format and the ST.26 format.

A nucleic acid molecule as described in Daniel O'Reilly et al., Nucleic Acids Research, 2019, Vol. 47, No.2, 546-558 (refer to p. 553) is exemplified. Typical examples thereof include a nucleic acid molecule having the following sequence (C).
Sequence (C): 5'-AGAGCCAGCCUUCUUAUUGUUUUAGAGCUAUGCUGU-3' (conforms to ST.25 format) (5'-AGAGCCAGCCTTCTTATTGTTTTAGAGCTATGCTGT-3' (conforms to ST.26 format)) (Sequence No. 3) 36 mer

A nucleic acid molecule having the following sequence (D) as described in Nucleic Acids Research, 2019, Vol. 47, No. 2: 547 is exemplified.
Sequence (D): 5'-ACAGCAUAGCAAGUUAAAAUAAGGCUAGUCCGUUAUCAACUUGAAAAAGUGGCACCGAG UCGGUGCU-3' (conforms to ST.25 format) ((5'-ACAGCATAGCAAGTTAAAATAAGGCTAGTCCGTTATCAACTTGAAAAAGTGGCACCGAG TCGGTGCT-3' (conforms to ST.26 format)) (Sequence No. 4) 67 mer

A nucleic acid molecule as described in JP 2015-523856 A1 page 173 is exemplified. Typical examples thereof include a nucleic acid molecule having the following sequence (E).
Sequence (E): 5'-GUUUUCCCUUUUCAAAGAAAUCUCCUGGGCACCUAUCUUCUUAGGUGCCCUCCCUUGUU UAAACCUGACCAGUUAACCGGCUGGUUAGGUUUUU-3' (conforms to ST.25 format) ((5'-GTTTTCCCTTTTCAAAGAAATCTCCTGGGCACCTATCTTCTTAGGTGCCCTCCCTTGTT TAAACCTGACCAGTTAACCGGCTGGTTAGGTTTT-3' (conforms to ST.26 format)) (Sequence No. 5) 94 mer

Nucleic acid molecules as described in JP 2017-537626 A1 are exemplified. Typical examples thereof include nucleic acid molecules having the following sequences (F), (G), (H) and (I).
Sequence (F): 5'-AGUCCUCAUCUCCCUCAAGCGUUUUAGAGCUAGUAAUAGCAAGUUAAAAUAAGGCUAGU CCGUUAUCAACUUGAAAAAGUGGCACCGAGUCGGUGCUUUU-3' (conforms to ST.25 format) ((5'-AGTCCTCATCTCCCTCAAGCGTTTTAGAGCTAGTAATAGCAAGTTAAAATAAGGCTAGT CCGTTATCAACTTGAAAAAGTGGCACCGAGTCGGTGCTTTT-3' (conforms to ST.26 format)) (Sequence No. 6) 100 mer
Sequence (G): 5'-GCAGAUGUAGUGUUUCCACAGUUUAAGAGCUAUGCUGGAAACAGCAUAGCAAGUUUAAA UAAGGCUAGUCCGUUAUCAACUUGAAAAAGUGGCACCGAGUCGGUGCUUUUUUU-3' (conforms to ST.25 format) ((5'-GCAGATGTAGTGTTTCCACAGTTTAAGAGCTATGCTGGAAACAGCATAGCAAGTTTAAA TAAGGCTAGTCCGTTATCAACTTGAAAAAGTGGCACCGAGTCGGTGCTTTTTTT-3' (conforms to ST.26 format)) (Sequence No. 7) 113 mer
Sequence (H): 5'-dAdGdTdCdCdTdCdAdTdCdTdCdCdCdTdCdAdAdGdCGUUUAAGAGCUAUGCUGGU AACAGCAUAGCAAGUUUAAAUAAGGCUAGUCCGUUAUCAACUUGAAAAAGUGGCACCGA GUCGGUGCUUUUUUU-3' (conforms to ST.25 format) ((5'-dAdGdTdCdCdTdCdAdTdCdTdCdCdCdTdCdAdAdGdCGTTTAAGAGCTATGCTGGT AACAGCATAGCAAGTTTAAATAAGGCTAGTCCGTTATCAACTTGAAAAAGTGGCACCGA GTCGGTGCTTTTTTT-3' (conforms to ST.26 format) (Sequence No. 8) 113 mer

In the sequence (H), dT represents thymidine, dC represents 2'-deoxycytidine, dA represents 2'-deoxyadenosine, and dG represents 2'-deoxyguanosine.
Sequence (I): 5'-AmsGmsUmsCCUCAUCUCCCUCAAGCGUUUAAGAGCUAUGCUGGUAACAGCAUAGCAAG UUUAAAUAAGGCUAGUCCGUUAUCAACUUGAAAAAGUGGCACCGAGUCGGUGCUUUUms UmsUmsU-3' (conforms to ST.25 format) ((5'-AmsGmsTmsCCTCATCTCCCTCAAGCGTTTAAGAGCTATGCTGGTAACAGCATAGCAAG TTTAAATAAGGCTAGTCCGTTATCAACTTGAAAAAGTGGCACCGAGTCGGTGCTTTTms TmsTmsT-3' (conforms to ST.26 format)) (Sequence No. 9) 113 mer

In the sequence (I), "Um" represents 2'-O-methyluridine (ST.25 format), "Tm" represents 2'-O-methyluridine (ST.26 format), "Am" represents 2'-O-methyladenosine, "Gm" represents 2'-O-methylguanosine, and "s" represents phosphorothioate modification.

### EXAMPLES

Hereinafter, the present invention is explained in more detail by working examples, but the present invention is not limited to these examples.

### Measurement methods

First, various measurement methods used in the following tests are shown below.

The purity of the oligonucleotide was measured using HPLC.

The HPLC measurement conditions are shown in Table 1 and Table 2 below.

### (Measurement method 1: Measurement of the purity of the oligonucleotide)

### [Table 1]

**Table 1**

| Column | Develsil ODS-UG-5 (4.6mm × 250mm, 5µm) |
|---|---|
| Flow rate | 1.0mL/min |
| Detection wavelength | 260nm |
| Mobile phase A | 10mM Ammonium acetate aqueous solution |
| Mobile phase B | Methanol/10mM Ammonium acetate aqueous solution mixture (1:1) |
| Gradient | B conc (%) 15%(0-3min)-25%(10min)-100%(20min) -100%(25min)-15%(25.01min)-15%(30min) |
| Column temperature | 30°C |

### (Measurement method 2: Measurement of the purity of the oligonucleotide)

### [Table 2]

**Table 2**

| Column | ACQUITY UPLC Oligonucleotide BEH C18 (2.1mm × 100mm, 1.7µm) |
|---|---|
| Flow rate | 0.2mL/min |
| Detection wavelength | 260nm |
| Mobile phase A | 100mM Hexylamine acetate aqueous solution (pH = 7.0) |
| Mobile phase B | 100mM Hexylamine acetate aqueous solution/Acetonitrile mixture (1:4) |
| Gradient | B conc (%) 43%(0min)-56%(70min)-90%(70.01min) -90%(75min)-43%(75.01min)-43%(90min) |
| Column temperature | 80°C |

### (Measurement method 3: Measurement of the ADTT residual rate)

ADTT in the thiolation solution was measured using HPLC.

The HPLC measurement conditions are shown in Table 3 below.

### [Table 3]

**Table 3**

| Column | YMC-Pack ODS-AQ (4.6mm × 250mm, 5µm) |
|---|---|
| Flow rate | 1.0mL/min |
| Mobile phase A | 10mM Ammonium acetate aqueous solution |
| Mobile phase B | Acetonitrile |
| Gradient | B conc (%) 15%(0min)-45%(30min)-90%(30.1min) -90%(40min)-15%(40.1min)-15%(55min) |
| Detection wavelength | 285nm |
| Column temperature | 25°C |

### Preparation of the thiolation solutions

The thiolation solutions used in the following Examples 15 to 19 were prepared as described in Examples 1 to 4, respectively. The ADTT residual rate after storing the prepared thiolation solutions at 50°C for 6 days is shown in Table 5. The ADTT residual rate was calculated based on the peak area value of ADTT contained in the thiolation solution immediately after preparation.

### Example 1

To 37.5 mg of commercially available ADTT, 12.2 g of pyridine and 10.8 g of toluene were added to dissolve the ADTT, and a thiolation solution was prepared. This solution was stored at 50°C for 6 days, and using the method described in the Measurement method 3, the ADTT residual rate was measured, resulting in a residual rate of 96%. The results are shown in Table 4. The solution stored at 50°C for 6 days was used in Example 15 and Example 19.

### Comparative Example 1

To 37.5 mg of commercially available ADTT, 12.2 g of pyridine and 9.8 g of acetonitrile were added to dissolve the ADTT, and a thiolation solution was prepared. This solution was stored at 50°C for 6 days, and using the method described in the Measurement method 3, the ADTT residual rate was measured, resulting in a residual rate of 28%. The results are shown in Table 4. The solution stored at 50°C for 6 days was used in Comparative Example 2.

### Example 2

To 37.5 mg of commercially available ADTT, 12.2 g of pyridine and 16.3 g of o-dichlorobenzene were added to dissolve the ADTT, and a thiolation solution was prepared. This solution was stored at 50°C for 6 days, and using the method described in the Measurement method 3, the ADTT residual rate was measured, resulting in a residual rate of 91%. The results are shown in Table 4. The solution stored at 50°C for 6 days was used in Example 16.

### Example 3

To 37.5 mg of commercially available ADTT, 12.2 g of pyridine and 11.0 g of o-xylene were added to dissolve the ADTT, and a thiolation solution was prepared. This solution was stored at 50°C for 6 days, and using the method described in the Measurement method 3, the ADTT residual rate was measured, resulting in a residual rate of 98%. The results are shown in Table 4. The solution stored at 50°C for 6 days was used in Example 17.

### Example 4

To 37.5 mg of commercially available ADTT, 11.5 g of 2,6-lutidine and 10.8 g of toluene were added to dissolve the ADTT, and a thiolation solution was prepared. This solution was stored at 50°C for 6 days, and using the method described in the Measurement method 3, the ADTT residual rate was measured, resulting in a residual rate of 93%. The results are shown in Table 4. The solution stored at 50°C for 6 days was used in Example 18.

### Example 5

To 37.5 mg of commercially available ADTT, 19.6 g of pyridine and 4.3 g of toluene were added to dissolve the ADTT, and a thiolation solution was prepared. This solution was stored at 50°C for 6 days, and using the method described in the Measurement method 3, the ADTT residual rate was measured, resulting in a residual rate of 88%. The results are shown in Table 5.

### Example 6

To 37.5 mg of commercially available ADTT, 16.3 g of pyridine and 7.2 g of toluene were added to dissolve the ADTT, and a thiolation solution was prepared. This solution was stored at 50°C for 6 days, and using the method described in the Measurement method 3, the ADTT residual rate was measured, resulting in a residual rate of 94%. The results are shown in Table 5.

### Example 7

To 37.5 mg of commercially available ADTT, 12.2 g of pyridine and 10.8 g of toluene were added to dissolve the ADTT, and a thiolation solution was prepared. This solution was stored at 50°C for 6 days, and using the method described in the Measurement method 3, the ADTT residual rate was measured, resulting in a residual rate of 96%. The results are shown in Table 5.

### Example 8

To 37.5 mg of commercially available ADTT, 8.2 g of pyridine and 14.3 g of toluene were added to dissolve the ADTT, and a thiolation solution was prepared. This solution was stored at 50°C for 6 days, and using the method described in the Measurement method 3, the ADTT residual rate was measured, resulting in a residual rate of 99%. The results are shown in Table 5.

### Example 9

To 37.5 mg of commercially available ADTT, 4.9 g of pyridine and 17.3 g of toluene were added to dissolve the ADTT, and a thiolation solution was prepared. This solution was stored at 50°C for 6 days, and using the method described in the Measurement method 3, the ADTT residual rate was measured, resulting in a residual rate of 100%. The results are shown in Table 5.

### Example 10

To 375 mg of commercially available ADTT, 12.2 g of pyridine and 10.8 g of toluene were added to dissolve the ADTT, and a thiolation solution was prepared. This solution was stored at 50°C for 6 days, and using the method described in the Measurement method 3, the ADTT residual rate was measured, resulting in a residual rate of 72%. The results are shown in Table 6.

### Example 11

To 188 mg of commercially available ADTT, 12.2 g of pyridine and 10.8 g of toluene were added to dissolve the ADTT, and a thiolation solution was prepared. This solution was stored at 50°C for 6 days, and using the method described in the Measurement method 3, the ADTT residual rate was measured, resulting in a residual rate of 75%. The results are shown in Table 6.

### Example 12

To 94.2 mg of commercially available ADTT, 12.2 g of pyridine and 10.8 g of toluene were added to dissolve the ADTT, and a thiolation solution was prepared. This solution was stored at 50°C for 6 days, and using the method described in the Measurement method 3, the ADTT residual rate was measured, resulting in a residual rate of 90%. The results are shown in Table 6.

### Example 13

To 37.9 mg of commercially available ADTT, 12.2 g of pyridine and 10.8 g of toluene were added to dissolve the ADTT, and a thiolation solution was prepared. This solution was stored at 50°C for 6 days, and using the method described in the Measurement method 3, the ADTT residual rate was measured, resulting in a residual rate of 98%. The results are shown in Table 6.

### Example 14

To 18.6 mg of commercially available ADTT, 12.2 g of pyridine and 10.8 g of toluene were added to dissolve the ADTT, and a thiolation solution was prepared. This solution was stored at 50°C for 6 days, and using the method described in the Measurement method 3, the ADTT residual rate was measured, resulting in a residual rate of 100%. The results are shown in Table 6.

### <Solid-phase synthesis of 2mer oligonucleotides>

Sequence (J):
5'-UmsUm-3' (ST.25 format) (5'-TmsTm-3' (ST.26 format)) (Sequence No. J)
In the sequence (J), "Ums" at the 5' terminal is represented by the upper substructure separated by a wavy line in the following formula (A1). "Um" at the 3' terminal is represented by the lower substructure separated by a wavy line in the following formula (A2).

### <Solid-phase synthesis of 100mer oligonucleotides>

Sequence (K): 5'-AmsCmsUmsCAAUUUGUAAAAAAGUUUUAGAGCUAGAAAUAGCAAGUUAAAAUAAGGCU AGUCCGUUAUCAACUUGAAAAAGUGGCACCGAGUCGGUGCUUUUmsUmsUmsU-3' (conforms to ST.25 format) (5'-AmsCmsTmsCAATTTGTAAAAAAGTTTTAGAGCTAGAAATAGCAAGTTAAAATAAGGCT AGTCCGTTATCAACTTGAAAAAGTGGCACCGAGTCGGTGCTTTTmsTmsTmsT-3' (conforms to ST.26 format)) (Sequence No. 10) 100mer

In the sequence (K), "A" is represented by the substructure delineated between the wavy lines in the following formula (A3). "C" is represented by the substructure delineated between the wavy lines in the following formula (A4). "G" is represented by the substructure delineated between the wavy lines in the following formula (A5). "U" is represented by the substructure delineated between the wavy lines in the following formula (A6). "Ams" is represented by the substructure delineated between the wavy lines in the following formula (A7) . "Cms" is represented by the substructure delineated between the wavy lines in the following formula (A8). "Ums" is represented by the substructure delineated between the wavy lines in the following formula (A9). Additionally, "Ams" at the 5' terminal is represented by the upper substructure delineated between the wavy lines in the following formula (A10). Furthermore, "U" at the 3' terminal is represented by the lower substructure delineated between the wavy lines in the following formula (A11).

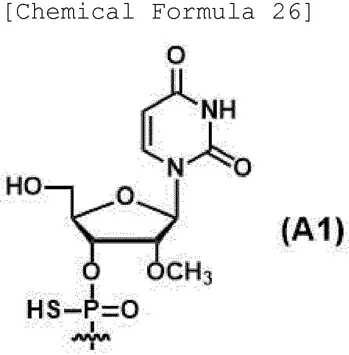

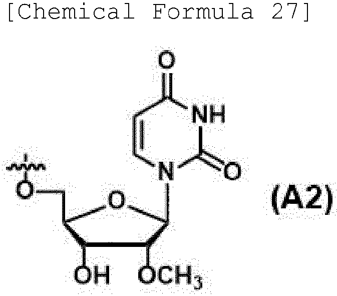

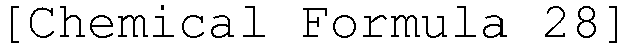

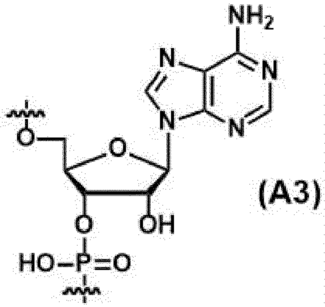

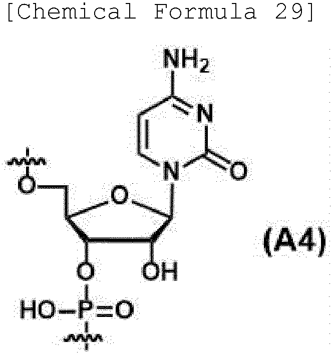

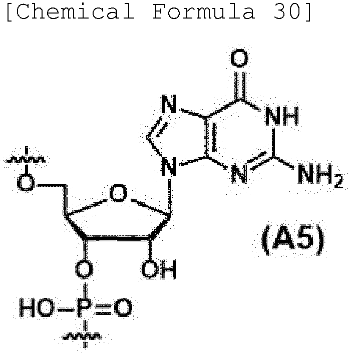

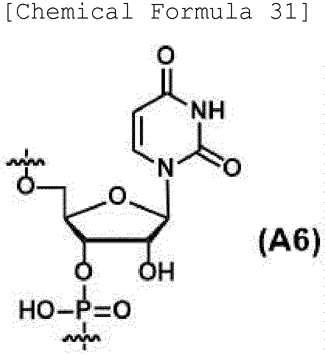

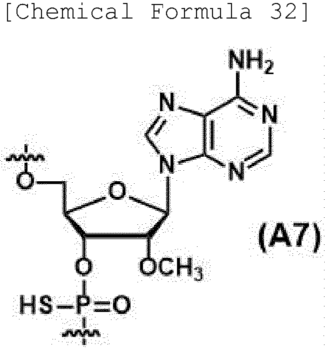

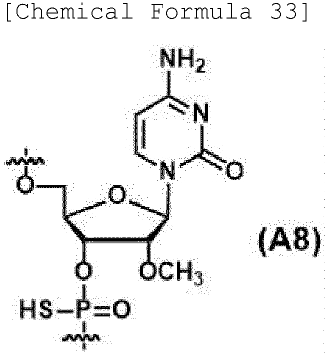

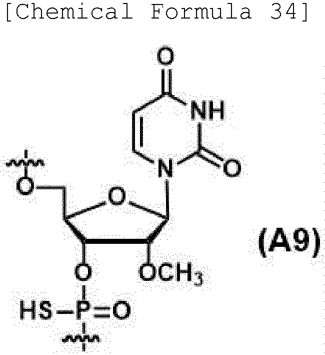

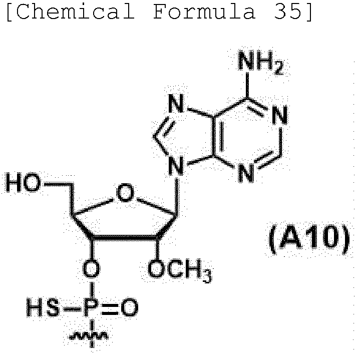

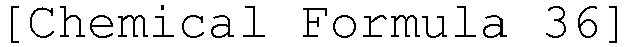

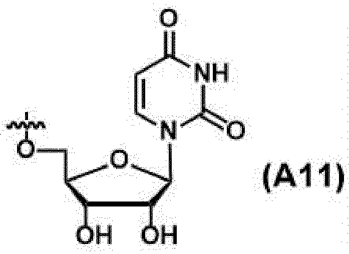

2'-OMe amidites and PMM amidites described in WO 2019/208571 A1 were used in the synthesis. Here, PMM stands for (((1-cyanopropan-2-yl)oxy)methoxy)methyl group.

2'-OMe uridine derivative (A12) and uridine derivative (A13) described in the following Examples and Comparative Examples refers to compounds represented by the following formulas. The circle depicted in the following formulas schematically represents CPG.

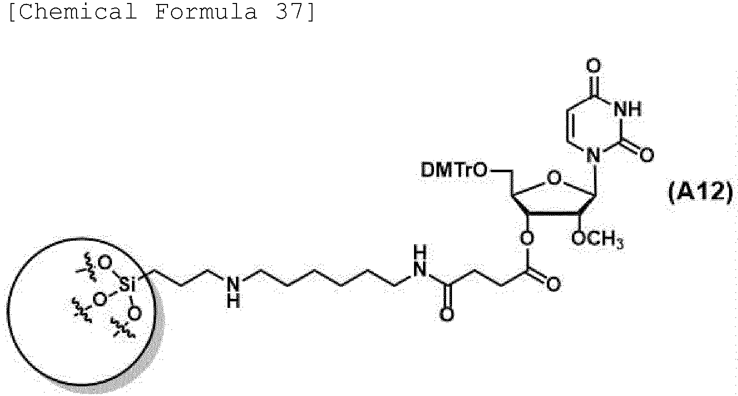

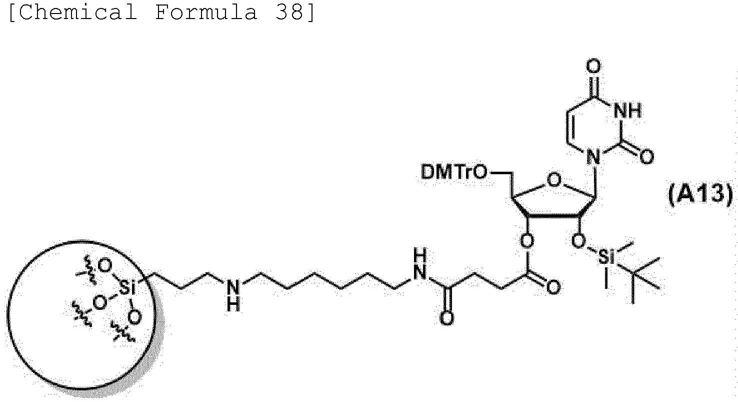

### (Example 15)

Using Controlled Pore Glass (CPG) loaded with 1.0 µmol of 2'-OMe uridine derivative, and 2'-OMe uridine amidite, an oligonucleotide composed by the sequence No. J (Sequence (J)) was automatically synthesized from the 3' side to the 5' side using the NTS M-4MX-E (manufactured by Nihon Techno Service Co., Ltd.). The procedure of automatic synthesis first involved liquid-transferring a 3% dichloroacetic acid toluene solution to the CPG to deprotect a trityl protecting group at the 5' position. Subsequently, 2'-OMe uridine amidite and 5-benzylmercapto-1H-tetrazole as a coupling agent were liquid-transferred to the CPG to proceed with the coupling reaction at the 5' hydroxy group. Subsequently, 100 µL of the thiolation solution prepared in Example 1 was added and left to stand for 16 minutes to convert a phosphite triester into a thiophosphate triester. Subsequently, the protecting group (DMTr group) of 2'-OMe uridine at the 5' terminal was deprotected using a 3% dichloroacetic acid toluene solution, and an oligonucleotide of Sequence No. 12 was synthesized on the CPG support. After that, 1.5 mL of 28% aqueous ammonia and 0.5 mL of ethanol were added to the CPG support loaded with 1.0 µmol of the oligonucleotide. The resulting mixture was incubated at 40°C for 4 hours to release the nucleic acid molecule from the solid support, and then the solvent was removed by concentration. The obtained crude product was dissolved in water, and the purity of the oligonucleotide was measured using the method described in Measurement method 1, and the purity was 58%. The results are shown in Table 7.

### (Comparative Example 2)

A nucleic acid molecule was obtained using the same method in the experiment of Example 15, except that the thiolation solution prepared in Comparative Example 1 was used as the thiolation solution. The purity of the oligonucleotide was measured using the method described in Measurement method 1, and the purity of the crude product was 23%. The results are shown in Table 7.

### (Example 16)

A nucleic acid molecule was obtained using the same method in the experiment of Example 15, except that the thiolation solution prepared in Example 2 was used as the thiolation solution. The purity of the oligonucleotide was measured using the method described in Measurement method 1, and the purity of the crude product was 52%. The results are shown in Table 7.

### (Example 17)

A nucleic acid molecule was obtained using the same method in the experiment of Example 15, except that the thiolation solution prepared in Example 3 was used as the thiolation solution. The purity of the oligonucleotide was measured using the method described in Measurement method 1, and the purity of the crude product was 52%. The results are shown in Table 7.

### (Example 18)

A nucleic acid molecule was obtained using the same method in the experiment of Example 15, except that the thiolation solution prepared in Example 4 was used as the thiolation solution. The purity of the oligonucleotide was measured using the method described in Measurement method 1, and the purity of the crude product was 55%. The results are shown in Table 7.

### (Example 19)

Using Controlled Pore Glass (CPG) loaded with 1.0 µmol of 2'-OMe uridine derivative, and 2'-OMe amidites and PMM amidites, an oligonucleotide composed by the sequence No. 10 (Sequence (K)) was automatically synthesized from the 3' side to the 5' side using the NTS M-4MX-E (manufactured by Nihon Techno Service Co., Ltd.). The procedure of automatic synthesis first involved liquid-transferring a 3% dichloroacetic acid toluene solution to the CPG to deprotect a trityl protecting group at the 5' position. Subsequently, 2'-OMe amidites or PMM amidites and 5-benzylmercapto-1H-tetrazole as a coupling agent were liquid-transferred to the CPG to proceed with the coupling reaction at the 5' hydroxy group. Subsequently, the thiolation solution prepared in Example 1 was liquid-transferred to convert a phosphite triester into a thiophosphate triester, or the oxidizing solution was liquid-transferred to convert a phosphite triester into a phosphate triester. Subsequently, using a 0.1 M phenoxyacetic anhydride acetonitrile solution and a 10 % *N*-methylimidazole/10 % 2,6-lutidine acetonitrile solution as capping solutions, capping was conducted at the reaction sites where the coupling reaction did not proceed. These steps were repeated a total of 99 times to synthesize an oligonucleotide of Sequence No. 13 on the CPG support. After that, a trityl protecting group at the 5' position was deprotected using a 3% dichloroacetic acid toluene solution. After that, 1.5 mL of 28% aqueous ammonia and 0.5 mL of ethanol were added to the CPG support loaded with 1.0 µmol of the oligonucleotide. The resulting mixture was incubated at 40°C for 4 hours to release the nucleic acid molecule from the solid support, and then the solvent was removed by concentration. After that, the released oligonucleotide was dissolved in 1.0 mL of dimethyl sulfoxide, followed by the addition of 13.5 µL of nitromethane and a stirring bar. Then, 2.0 mL of a 1 M tetrabutylammonium fluoride (TBAF) dimethyl sulfoxide solution, dehydrated using molecular sieves 4A, was added at 30°C under stirring with a stirrer. The resulting mixture was incubated for 5 hours to deprotect the protecting group of 2'-hydroxyl group. The oligonucleotide was obtained by precipitation. The obtained crude product was dissolved in water, and the purity of the oligonucleotide was measured using the method described in Measurement method 3, and the purity of the crude product was 40%. The results are shown in Table 8.

### [Table 4]

**Table 4**

| | Solvent Composition | ADTT residual rate (%) |
|---|---|---|
| Example 1 | pyridine-toluene | 96% |
| Comparative Example 1 | pyridine-acetonitrile | 28% |
| Example 2 | pyridine-*o*-dichlorobenzene | 91% |
| Example 3 | pyridine-*o*-xylene | 98% |
| Example 4 | 2,6-lutidine-toluene | 93% |

### [Table 5]

**Table 5**

| | Solvent Composition | ADTT residual rate (%) |
|---|---|---|
| Example 5 | pyridine-toluene | 88% |
| Example 6 | pyridine-toluene | 94% |
| Example 7 | pyridine-toluene | 96% |
| Example 8 | pyridine-toluene | 99% |
| Example 9 | pyridine-toluene | 100% |

### [Table 6]

**Table 6**

| | Solvent Composition | ADTT residual rate (%) |
|---|---|---|
| Example 10 | pyridine-toluene | 72% |
| Example 11 | pyridine-toluene | 75% |
| Example 12 | pyridine-toluene | 90% |
| Example 13 | pyridine-toluene | 98% |
| Example 14 | pyridine-toluene | 100% |

### [Table 7]

**Table 7**

| | Synthesized Sequence | Purity of the crude product (%) |
|---|---|---|
| Example 15 | Sequence No. J | 58% |
| Comparative Example 2 | Sequence No. J | 23% |
| Example 16 | Sequence No. J | 52% |
| Example 17 | Sequence No. J | 52% |
| Example 18 | Sequence No. J | 55% |

### [Table 8]

**Table 8**

| | Synthesized Sequence | Purity of the crude product (%) |
|---|---|---|
| Example 19 | Sequence No. 2 | 40% |

From the results in Tables 4 to 6, Examples 1 to 14 using the thiolation solution of the present invention showed higher ADTT residual rates compared to the thiolation solution of Comparative Example 1.

From the results in Tables 7 to 8, Examples 15 to 19 using the thiolation solution of the present invention yielded nucleic acid molecules with higher purity compared to the case where the thiolation solution of Comparative Example 2 was used.

### INDUSTRIAL APPLICABILITY

The present invention provides a thiolation solution with high stability, which contains a compound represented by formula (3) (i.e., ADTT) as a thiolating agent, and a production method of a nucleic acid molecule by the amidite method using the thiolation solution. The present invention is expected to improve the stability of a thiolation solution. Additionally, the present invention is expected to improve the purity of the nucleic acid molecule produced.

### [Free text of Sequence Listing]

Sequence Nos. 1 to 10 in Sequence Listing represent base sequences of oligonucleotides that are produced according to the production method of the present invention.

[Sequence Listing]

## Claims

1. A composition comprising:
an aromatic heterocyclic compound represented by formula (1) : 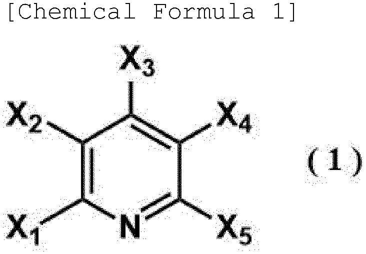 (wherein, X₁ to X₅ are identical to or different from each other and each independently represents a hydrogen atom or a C1-C5 alkyl group.),
an aromatic hydrocarbon compound represented by formula (2): 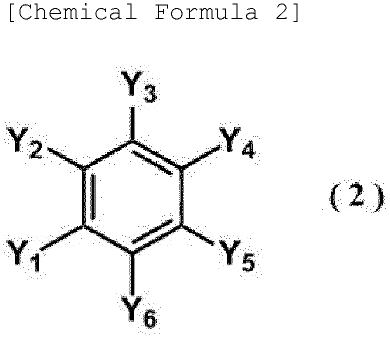 (wherein, Y₁ to Y₆ are identical to or different from each other and each independently represents a hydrogen atom, a C1-C5 alkyl group, or a halogen atom.), and
a thiolating agent represented by formula (3):

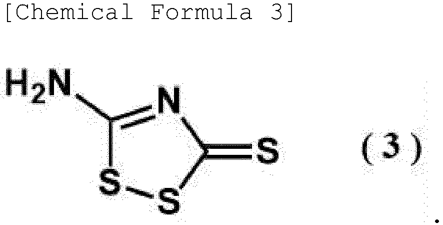

2. The composition according to claim 1, wherein the aromatic heterocyclic compound is pyridine, 2-methylpyridine, 3-methylpyridine, 4-methylpyridine, 2,6-lutidine, 3,4-lutidine, 3,5-lutidine, 2,3-lutidine, 2,4-lutidine, or 2,5-lutidine.

3. The composition according to claim 1, wherein the aromatic heterocyclic compound is pyridine or 2,6-lutidine.

4. The composition according to any one of claims 1 to 3, wherein the aromatic hydrocarbon compound is benzene, toluene, o-xylene, m-xylene, p-xylene, chlorobenzene, o-dichlorobenzene, m-dichlorobenzene, or p-dichlorobenzene.

5. The composition according to any one of claims 1 to 4, wherein the aromatic hydrocarbon compound is toluene, o-xylene, or o-dichlorobenzene.

6. The composition according to any one of claims 1 to 5, wherein the aromatic heterocyclic compound is pyridine.

7. The composition according to any one of claims 1 to 6, wherein the aromatic hydrocarbon compound is toluene.

8. The composition according to any one of claims 1 to 7, wherein the volume ratio of the aromatic heterocyclic compound to the aromatic hydrocarbon compound is 1:99 to 99:1.

9. The composition according to any one of claims 1 to 7, wherein the volume ratio of the aromatic heterocyclic compound to the aromatic hydrocarbon compound is 1:4 to 4:1.

10. The composition according to any one of claims 1 to 7, wherein the volume ratio of the aromatic heterocyclic compound to the aromatic hydrocarbon compound is 2:1 to 1:2.

11. The composition according to any one of claims 1 to 10, wherein the concentration of the thiolating agent represented by formula (3) in the composition is 0.001 to 0.3 M.

12. A production method of a nucleic acid molecule by the amidite method, comprising a step of converting a phosphite triester bond to a thiophosphate triester bond using the composition according to any one of claims 1 to 11.

13. A production method of a nucleic acid molecule comprising a step of contacting a precursor represented by formula (4): 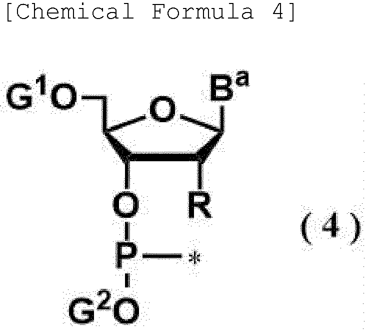 (wherein,
G¹ and G² are identical to or different from each other and each independently represents a protecting group of a hydroxy group,
B^{a} represents a nucleobase which may be protected by a protecting group,
R represents a protected hydroxy group, a hydrogen atom, a fluorine atom, a methoxy group, a 2-methoxyethyl group, an OQ' group, or an NQ' group,
Q' represents an alkylene group or a carbonyl group , which are bonded to the carbon atom at the 4' position of the ribose, and,
the bond marked with * indicates the binding position to the nucleotide unit at the 3' terminal side.) which has a phosphite triester bond with the composition according to any one of claims 1 to 12 to convert into a nucleic acid compound represented by formula (5): 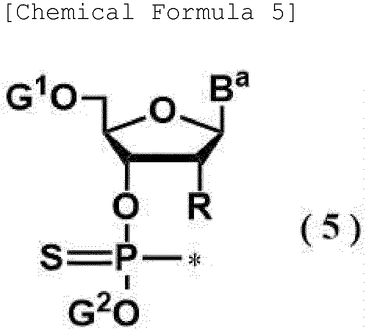 (wherein, the symbols have the same meanings as described above.)
which has a thiophosphate triester bond.

14. The production method of the nucleic acid molecule according to claim 13, wherein the precursor which has the phosphite triester bond is a compound represented by formula (6) : 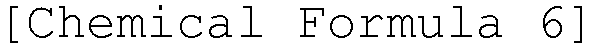 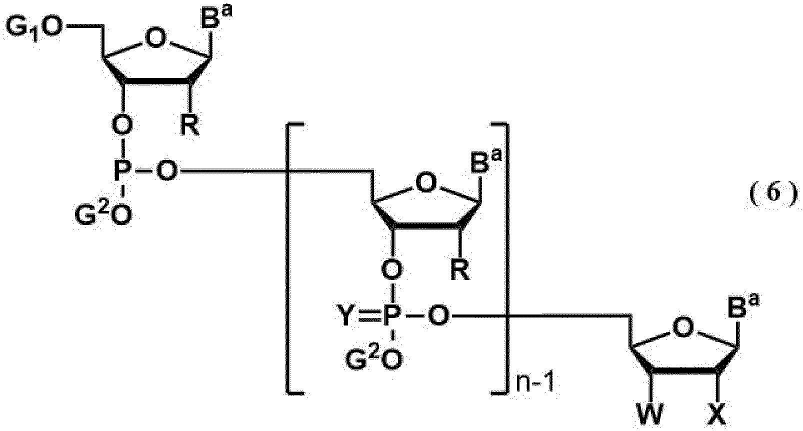 (wherein,
G¹ represents a protecting group of a hydroxy group,
G² is identical to or different from each other and each independently represents a protecting group of a hydroxy group,
B^{a} is identical to or different from each other and each independently represents a nucleobase which may be protected by a protecting group,
R is identical to or different from each other and each independently represents a protected hydroxy group, a hydrogen atom, a fluorine atom, a methoxy group, a 2-methoxyethyl group, an OQ' group, or an NQ' group,
Q' is identical to or different from each other and each independently represents an alkylene group or a carbonyl group, which are bonded to the carbon atom at the 4' position of the ribose,
Y is identical to or different from each other and each independently represents an oxygen atom or a sulfur atom,
n represents any integer from 1 to 300,
when X represents an OZ group, W represents an OV group, and V represents a protecting group of a hydroxy group, or,
when X represents an R group, W represents an OZ group, and,
Z is a group having a structure consisting of a solid support and a connecting group.), and, the compound which has the thiophosphate triester bond is a nucleic acid compound represented by formula (7): 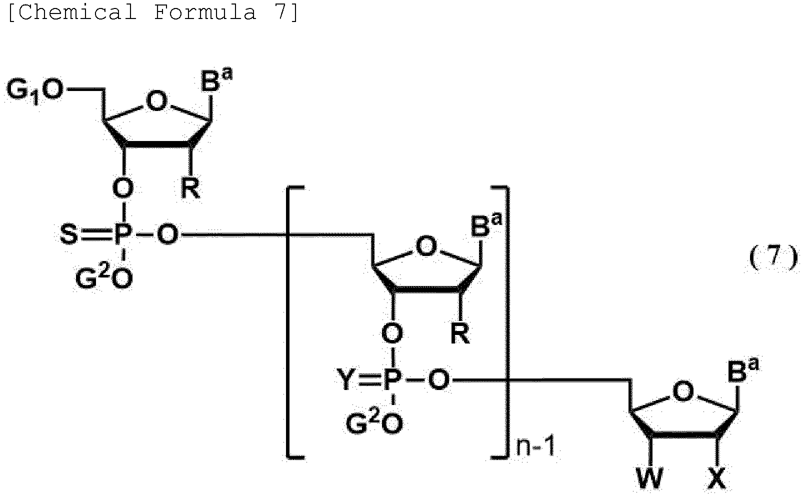 (wherein, the symbols have the same meanings as described above.) .

15. The production method of the nucleic acid molecule according to claim 13 or 14, wherein the precursor which has the phosphite triester bond is a compound represented by formula (8): 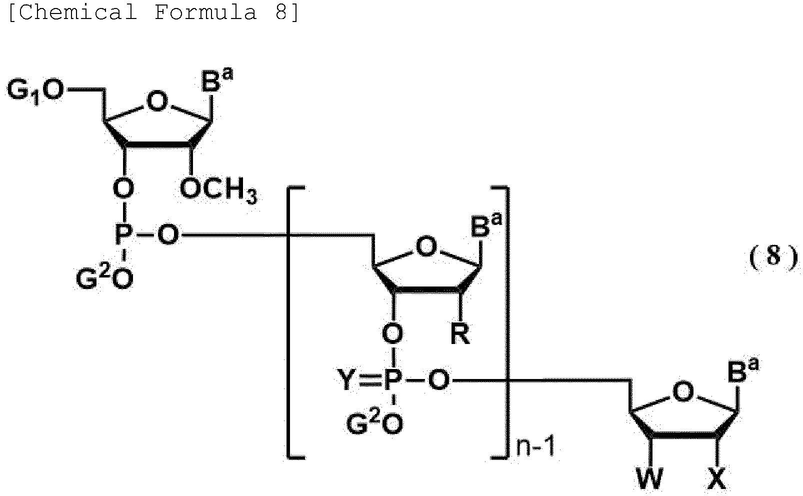 (wherein,
G¹ represents a protecting group of a hydroxy group,
G² is identical to or different from each other and each independently represents a protecting group of a hydroxy group,
B^{a} is identical to or different from each other and each independently represents a nucleobase which may be protected by a protecting group,
R is identical to or different from each other and each independently represents a protected hydroxy group, a hydrogen atom, a fluorine atom, a methoxy group, a 2-methoxyethyl group, an OQ' group, or an NQ' group,
Q' is identical to or different from each other and each independently represents an alkylene group or a carbonyl group, which are bonded to the carbon atom at the 4' position of the ribose,
Y is identical to or different from each other and each independently represents an oxygen atom or a sulfur atom,
n represents any integer from 1 to 300,
when X represents an OZ group, W represents an OV group, and V represents a protecting group of a hydroxy group, or,
when X represents an R group, W represents an OZ group, and,
Z is a group having a structure consisting of a solid support and a connecting group.), and, the compound which has the thiophosphate triester bond is a nucleic acid compound represented by formula (9): 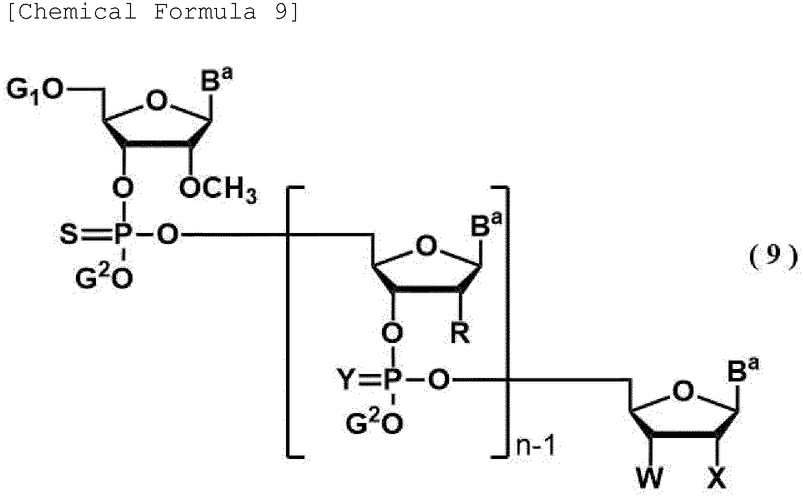 (wherein, the symbols have the same meanings as described above.) .
